# EUROPEAN PATENT SPECIFICATION

(11) **EP 0 574 576 B1**
(45) Date of publication and mention of the grant of the patent: **26.03.1997**
(21) Application number: 93902963.3
(22) Date of filing: 07.01.1993
(51) Int. Cl.: A61F 2/16, B29D 11/00

(54) **MOLDED ARTICLE AND METHOD AND APPARATUS FOR MOLDING THE ARTICLE**
FORMTEIL SOWIE VERFAHREN UND VORRICHTUNG ZUM FORMEN DES TEILS
ARTICLE MOULE ET PROCEDE ET APPAREIL DE MOULAGE DE L'ARTICLE

(30) Priority: 08.01.1992 US 818074
(43) Date of publication of application: 22.12.1993
(73) Proprietor: PY, Daniel, Wellesley, MA 02181 (US)
(72) Inventor: PY, Daniel, Wellesley, MA 02181 (US)
(74) Representative: Robinson, Nigel Alexander Julian
(86) International application number: US9300085
(87) International publication number: WO9313731

(56) References cited:
- US-A- 4 865 601
- PATENT ABSTRACTS OF JAPAN vol. 012, no. 296 (M-731)12 August 1988 & JP-A-63 074 617 (AKINOBU TATEBAYASHI)

## Description

### Field of the Invention

The present invention relates to methods and apparatus for molding articles and, more particularly, to methods and apparatus for molding polymeric materials into articles such as prosthetic devices requiring a porous interface for anchoring the devices to living tissue.

### Background Information

There are various known prostheses designed for the replacement of diseased or injured corneas. One such prosthesis is shown in U.S. Patent No. 4,865,601, which is intended to lie deep in an intra-lamellar stromal pocket and to be anchored in place by a fibrous ingrowth of tissue into an annular porous skirt. The prosthesis includes an optical element made from polyurethane, which is surrounded by a porous annular skirt made from polytetrafluoroethylene ("Goretex"). The porous skirt is intended to permit the fibrous ingrowth of tissue. The optical element is attached to the annular skirt by introducing a polymerization mixture into the area where the optical element and skirt overlap, to bond the two different materials together.

Because the optical element and the skirt are made of different materials, it is inherently difficult to bond the two components together. Thus, once implanted in an eye, the bond between the optical element and the skirt is subject to degradation and failure. This difficulty has also been encountered with other types of prostheses in which it is desirable to have a porous portion to permit the ingrowth of living tissue to anchor the prosthesis to the patient's body, and to have another portion of the prosthesis which is not porous in order to retain fluid within the patient's body, for example. Both the porous and the non-porous materials must be biocompatible, and must be attached to one another to form the prosthesis in a manner which will not degrade or otherwise fail upon being implanted in a patient's body.

It has proven to be particularly difficult to attach such a porous portion to a non-porous polymeric portion in a manner which will not rapidly degrade upon implantation in living tissue. Known methods of ultrasonic welding or high frequency welding have not proven to be satisfactory for attaching such polymeric materials, particularly because the soft porous structure of the porous portion tends to reduce the vibrations generated by the welding process within the material, and thus prevents the necessary heat from being generated in order to weld the materials together. Known methods of chemically bonding have also proven to be generally unsatisfactory. The substances used to chemically bond these materials vary with the types of polymeric materials used, and in many instances these chemical substances are toxic when not combined with other polymeric materials. This can present a serious safety hazard, particularly if any residual toxic material is captured within the pores of the porous portion, since such toxic material can provoke major inflammation which can in turn prevent a sufficient ingrowth of living tissue into the porous portion to properly anchor the prosthesis in place.

### Summary of the Invention

The present invention is directed to a method of moulding a polymeric article including a solid polymeric portion, said solid polymeric portion forming a prosthetic lens, and a porous polymeric portion comprising the steps of placing the porous polymeric portion within a die cavity and introducing a liquid polymer into the die cavity for forming the prosthetic lens and for flowing into at least a portion of the pores of the porous polymeric portion to form an integral solid portion and porous portion upon cooling of the liquid polymer.

In one embodiment of the present invention, the polymeric article is an ocular prosthesis and the porous portion forms a skirt surrounding the lens for permitting the ingrowth of living tissue into the pores of the skirt upon implantation in an eye. The method of the present invention preferably further comprises the step of compressing a portion of the porous portion upon introducing the liquid polymer into the die cavity to permit the liquid polymer to flow into the pores of a limited area of the porous portion so that the remainder of the porous portion remains porous. In one embodiment of the present invention, the liquid polymer is a polyether-based polyurethane, and the porous portion is made from a polyester or a polyether-based polyurethane.

In this embodiment of the present invention, the method preferably further comprises the step of cooling a surface defining the die cavity upon introducing the liquid polymer into the die cavity to facilitate obtaining high optical resolution with the lens. The method also preferably further comprises the step of heating the liquid polymer immediately prior to introduction into the die cavity to reduce the viscosity of the liquid polymer upon introduction into the die cavity.

The present invention is also directed to a prosthesis for implantation within living tissue, comprising a solid polymeric portion forming a prosthetic lens, and an integral porous polymeric portion. The prothesis is formed by introducing the polymer of the solid portion as a heated liquid into the pores of at least a portion of the porous portion and permitting the liquid polymer to cool and simultaneously form the solid portion integral with the porous portion. The porous portion is provided to permit the ingrowth of living tissue upon implantation of the prosthesis.

In one embodiment of the present invention, the prosthesis is an ocular prosthesis and the porous portion forms a skirt surrounding the lens for permitting the ingrowth of living tissue into the pores of the skirt upon implantation in an eye. In this embodiment, the solid portion is preferably made from a polyether-based polyurethane, and the porous portion is preferably made from a polyester or polyether-based polyurethane.

The present invention is also directed to an apparatus for moulding a polymeric article including a solid polymeric portion, said solid polymeric portion forming a prosthetic lens, and a porous polymeric portion, comprising:
a first die for receiving said porous portion of the article:
a second die movable relative to the first die wherein the first and second dies define a die cavity therebetween;
a means for conveying a liquid polymer to the cavity, the cavity forming said solid portion of the article upon cooling of the liquid polymer within the die cavity and permitting a portion of the liquid polymer to flow into at least a portion of the pores of the porous portion of the article to form an integral porous portion and solid portion upon cooling of the liquid polymer; and
a third die movable relative to the first die and radially disposed about the second die for securing the porous portion of the article in position.

The apparatus of the present invention preferably comprises a conduit coupled in fluid communication with the die cavity for introducing the liquid polymer into the die cavity. At least one heating element is located adjacent to a wall of the conduit for heating the liquid polymer immediately prior to introduction into the die cavity to reduce the viscosity of the liquid polymer upon introduction into the die cavity. A cooling element is also preferably located adjacent to a surface defining the die cavity for cooling the surface to prevent the formation of flow lines and granularity on the surface of the solid portion of the article.

One advantage of the present invention is that because the liquid polymer forming the solid portion is permitted to penetrate the porous polymer of the porous portion, the solid portion is formed as an integral portion of the porous portion. This is particularly advantageous for prostheses, because the integral structure of the solid and porous portions substantially prevents any degradation in this area upon implantation in living tissue.

Other advantages of the present invention will become apparent in view of the following detailed description and accompanying drawings.

### Brief Description of the Drawings

Figure 1 is a cross-sectional view of an apparatus embodying the present invention for molding articles in accordance with a method of the present invention and a cross-sectional view of an ocular prosthesis embodying the present invention.

Figure 2 is a plan view of the ocular prosthesis of Figure 1 made with of the apparatus of Figure 1 in accordance with a method of the present invention.

### Detailed Description

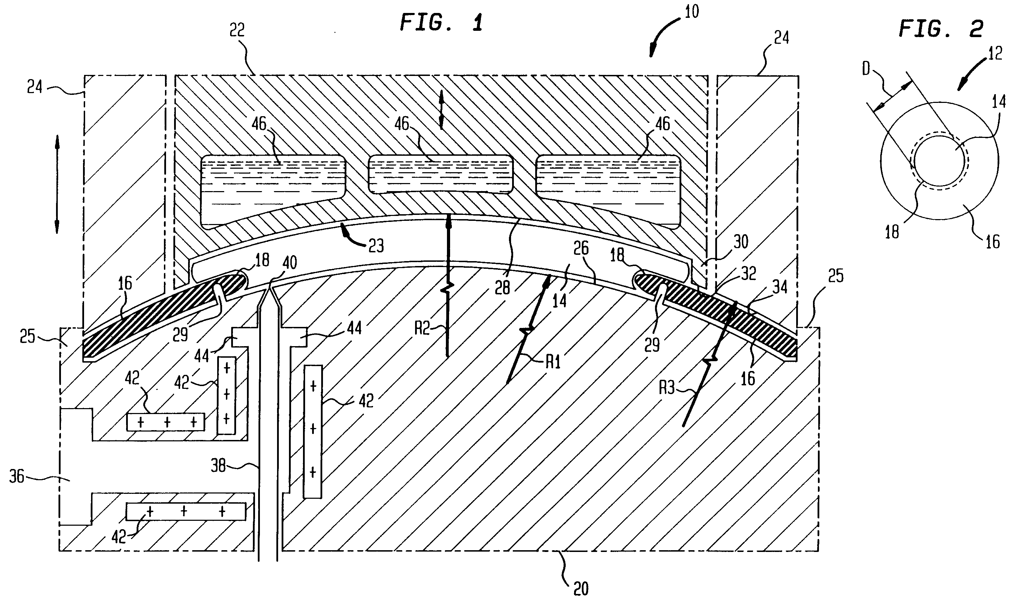

In Figure 1, an apparatus for molding polymeric materials into articles in accordance with a method of the present invention is indicated generally by the reference numeral 10. In the embodiment of the present invention illustrated, the apparatus 10 is employed to mold polymeric materials into an article which is an ocular prosthesis 12 illustrated typically in Figure 2. As will be recognized by those skilled in the art, however, the apparatus and method of the present invention are equally applicable for molding polymeric materials into numerous other types of articles.

The ocular prosthesis 12 includes a lens 14 and a skirt 16 surrounding the lens 14. The prosthesis 12 is typically used to replace a diseased or damaged cornea which has been surgically removed. The diameter D of the lens 14 is preferably equal to the normal cornea diameter. The prosthesis 12 is implanted in an eye by dissecting the peripheral cornea or scleral and inserting the skirt 16 to anchor the prosthesis to the living tissue of the eye, as is described further below. The lens 14 and skirt 16 are each made of a polymeric material which is biocompatible, inert, and non-biodegradable. The material for the lens is also optically clear and UV stable. One such material for the lens is the aliphatic, polyether-based polyurethanes, such as the Tecoflex medical grade polyurethane resins (Tecoflex grade EG80A, for example) available from Thermedics, Inc. of Woburn Massachusetts. The lens 14 can thus be essentially the same as the lens as described in US patent application serial no. 07/702,048, filed May 17, 1991, entitled "Ocular Prosthesis".

The skirt 16 is porous to facilitate the ingrowth of living tissue upon implantation into an eye to anchor the prosthesis 12 to the eye. The skirt 16 is preferably made from a porous polyester, such as Dacron polyester, which is commercially available from DuPont, and which is knitted or woven in a manner known to those of ordinary skill in the pertinent art to form a substantially uniform porous structure having pore dimensions on the order of approximately 100 microns. The pore dimensions can be varied, however, and are preferably within the range of approximately 50 to 600 microns. The skirt 16 can also be made from the polyether-based polyurethanes described above, which can be made porous by electrostatically spinning the polyurethane into porous sheets, as also described in the above-mentioned co-pending patent application.

The prosthesis 12 is made by employing the apparatus 10 which, as shown in Figure 1, includes a first die 20, a second die 22, and a third die 24 surrounding the second die 22. A mold cavity 23 is thus defined between the first die 20 and second die 22, and the second die 22 and third die 24 are both moveable relative to the first die 20, as indicated by the arrows in Figure 1. The prosthesis 12 is formed by initially cutting the skirt 16 as a single piece with a die cutter (not shown) from a sheet of porous polymer. The skirt 16 is then placed into the mold cavity 23, as shown in Figure 1. The second and third dies 22 and 24, respectively, are then moved toward the first die 20 to fix the skirt 16 in place, and liquid polymer is then injected into the mold cavity 23 to form the lens 14. The liquid polymer immediately fills the mold cavity to form the lens 14, and in turn penetrates the porous polymer of the skirt 16 in an overlap area 18 where the lens overlaps the skirt to form an integral skirt and lens, as is described further below.

The first die 20 defines a first die surface 26 which substantially conforms in shape to the posterior side of the lens 14 and skirt 16, and is defined by a radius of curvature R1 which is dimensioned to correspond to the radius of curvature of the posterior side of the prosthesis 12. Several pin members 29 project outwardly from the first die surface 26 and are spaced apart from each other along an annular path. As shown in Figure 1, the pin members 29 are provided for penetrating the overlap area 18 of the skirt 16 upon being placed into the apparatus 10 to hold the skirt in position during the molding process. The first die 20 also includes a peripheral flange 25 for guiding and receiving the second die 24 during the molding process.

The second die 22 defines a second die surface 28 spaced apart from the first die surface 26 a distance substantially equal to the thickness of the lens 14. The second die surface 28 corresponds in shape to the anterior side of the lens 14 and is defined by a radius of curvature R2. The second die 22 also includes a flange portion 30 surrounding the second die surface 28 for forming the peripheral edge of the lens 14. The flange portion 30 defines a third die surface 32 which is spaced apart from the first die surface 26 a distance approximately equal to the thickness of the skirt 16. The third die surface 32 is provided for compressing a peripheral portion of the skirt 16 surrounding the overlap area 18 as shown in Figure 1 to substantially close the pores of the porous polymer, and in turn prevent the injected liquid polymer from penetrating that portion of the skirt. The liquid polymer is thus permitted to penetrate the porous skirt substantially only in the overlap area 18, as shown in Figure 1, to form an integral skirt and lens, and the remaining portion of the skirt 16 remains porous to permit the ingrowth of living tissue upon implantation in the eye.

The third die 24 defines a fourth die surface 34, which surrounds the third die surface 32 and is spaced above the first die surface 26 a distance approximately equal to the thickness of the skirt 16. As shown in Figure 1, the fourth die surface 34 is defined by a radius of curvature R3 which is approximately the same as the radius of curvature of the third die surface 32. The third die 24 lightly compresses the adjacent portion of the skirt 16 during the molding process to prevent the penetration of liquid polymer into that portion of the skirt so that the portion of the skirt 16 surrounding the overlap area 18 remains porous to permit the ingrowth of living tissue upon implantation in the eye.

A barrel 36 is coupled to a typical injection molding machine (not shown) for injecting the liquid polymer into the mold cavity. A tunnel 38 is coupled on one end to the barrel 36 and is coupled on the other end to a tunnel gate 40 which opens through the first die surface 26. The liquid polymer is injected from the barrel 36, through the tunnel 38, and in turn through the tunnel gate 40 into the mold cavity to form the lens 14. The surfaces of the barrel 36 and tunnel 38 are heated, as indicated by several heating elements 42 illustrated schematically in Figure 1. Because in the illustrated embodiment the polyether-based polyurethane of the lens 14 is a relatively viscous material, these surfaces are heated to maintain the polyurethane at a higher temperature and thus at a lower viscosity upon injection through the tunnel gate 40 into the mold cavity 23. The liquid polyurethane is preferably injected at a temperature of approximately 182°C (360°F) and a pressure of approximately 1.8 mm mg. Receptacles 44 (referred to as "cold shots") are coupled in fluid communication with the tunnel 38 immediately prior to the tunnel gate 40 for receiving the polymer flowing in the region closer to the walls of the tunnel which might tend to cool down and thus form chips and/or granularize. The hottest polymer (and most homogeneous) is therefore injected through the tunnel gate 40 into the mold cavity, whereas the polymer that might tend to chip and/or granularize flows off into the receptacles 44.

The second die surface 28 is cooled by several cooling channels 46 extending through the second die 22 adjacent to the second die surface, as shown in Figure 1. In the embodiment of the present invention illustrated, water at about 18°C flows through the cooling channels 46 at a rate of about 10 liters per minute to cool the second die surface 28. The cooling of the second die surface 28 prevents the formation of flow lines and granularity on the surface of the lens 14, prevents warpage of the lens 14 upon removal from the mold cavity, and thus facilitates obtaining the best optical resolution and consistent quality.

In the operation of the apparatus and method of the present invention, a sheet of porous polymer, preferably approximately .25 mm thick, is cut with a die cutter (not shown) to form the skirt 16. The skirt 16 is then placed on the first die 20 so that its posterior side is seated on the first die surface 26 and the overlap area 18 is seated over the pin members 29. The second die 22 and third die 24 are then moved toward the first die 20 so that the third die surface 32 and the fourth die surface 34 are moved into engagement with the anterior side of the skirt 16. The second and third dies 22 and 24, respectively, are then moved toward the first die 20 a distance sufficient to cause the pin members 29 to penetrate the porous polymer of the overlap area 18 to fix the skirt 16 in place and to lightly compress the porous polymer to substantially prevent the penetration of the liquid polyurethane into the pores of the compressed porous polymer.

The liquid polymer is then injected through the barrel 36 and tunnel 38, and in turn through the tunnel gate 40 into the mold cavity 23. The heating elements 42 are elevated to a temperature sufficient to maintain the liquid polymer in the approximate central area of the tunnel 38 at a temperature of approximately 182°C (360° F), whereas the lower temperature polymer which might tend to form chips and/or granularize is permitted to flow into the receptacles 44. The hot liquid polymer is then injected at a pressure of about 1.8 mm mg through the tunnel gate 40 to fill the mold cavity and thus form the lens 14. The cooling channels 46 cool the second die surface 28 relative to the hot polymer to prevent the formation of flow lines and granularity on the surfaces of the lens 14, prevent warpage of the lens 14 upon removal from the mold, and to obtain the best optical resolution of the lens 14 and consistent quality. The liquid polymer in turn penetrates the porous polymer of the skirt 16 in the overlap area 18 to form an integral lens and skirt.

As will be recognized by those skilled in the art, it is imperative that the lens 14 and skirt 16 not degrade or otherwise fail upon implantation in the eye. One advantage of the present invention, is that because the liquid polymer along the peripheral portion of the lens 14 is permitted to penetrate the porous polymer of the skirt 16, the lens is formed as an integral part of the skirt, thus forming an integral prosthesis. The prosthesis of the present invention should thus prove to be significantly superior to prior prostheses in which the lens is bonded to the skirt by means of an adhesive or solvent solution.

With the prosthesis of the present invention, on the other hand, because the lens 14 is injection molded as an integral part of the skirt 16, the area where the lens joins the skirt should be as strong and durable as the other portions of the prosthesis. Accordingly, the safeness of an implantation can be significantly increased with a prosthesis made in accordance with the present invention.

The prosthesis 12 is implanted by removing the entire cornea at the limbus, including the peripheral germinative area, which is a highly active anabolic area of the corneal epithelium. The removal of the cornea at the limbus allows an incision to be made in the superficial layer of the sclera at a depth of about 3 mm to permit insertion of the porous skirt 16. Once implanted, the living tissue of the eye is permitted to grow into the pores of the skirt 16 to anchor the prosthesis 12 to the eye.

Downgrowth of the corneal epithelium occurs when the germinative layer of the corneal epithelium becomes rampant and invades the anterior chamber of the eye, which can occur through the slightest opening in the anterior chamber. This downgrowth of the corneal epithelium can cause the eye to reject a prosthesis and contribute to the degradation of a bond between a porous portion and a lens. As will be recognized by those skilled in the art, however, one advantage of the prosthesis of the present invention is that it substantially prevents such downgrowth by the removal of the entire cornea at the limbus and the germinative layer, and the integral structure of the skirt and lens substantially prevents any degradation of the prosthesis upon implantation. The prosthesis of the present invention thus facilitates an active cellular metabolism for the ingrowth of the living tissue into the porous skirt, including the cellular and plasmatic components of the blood supply which exist in the sclera. The prosthesis of the present invention thus facilitates a more rapid healing process upon implantation than with many prior prostheses.

Another advantage of the prosthesis of the present invention, is that upon implantation, the aqueous veins, which transport aqueous humor from the anterior chamber of the eye into the sub-conjunctival venous network, are destroyed. An outflow of this aqueous humor can lead to a post-surgical increase in the intra-ocular pressure, which can in turn lead to glaucoma. However, because the aqueous veins are destroyed upon implantation of the prosthesis of the present invention, such an outflow of aqueous humor is inhibited. Also, the porous structure of the skirt 16 of the prosthesis permits the passage of small quantities of the aqueous humor from the anterior chamber to the subconjunctival tissue, where the aqueous humor can be reabsorbed to further prevent a post-surgical increase in the intra-ocular pressure.

As will also be recognized by those skilled in the art, the apparatus and method of the present invention is not limited to the manufacture of ocular prostheses as shown in Figure 2, nor to the use of the particular polymeric materials described. Rather, the apparatus and method of the present invention is equally applicable for manufacturing numerous other types of prostheses and implantable devices which may require an integral structure formed from different biocompatible polymeric materials. For example, in numerous such devices it would be desirable to have a porous portion for permitting the ingrowth of tissue to anchor the device to the patient's body, and to have an integral non-porous portion for retaining fluids within the patient's body. Thus, the apparatus and method of the present invention can be employed to injection mold the non-porous portion as an integral part of the porous portion to form a safe and durable implantable device.

## Claims

1. A method of moulding a polymeric article (12) including a solid polymeric portion, said solid polymeric portion forming a prosthetic lens (14), and a porous polymeric portion (16), comprising the steps of placing the porous polymeric portion within a die cavity (23) and introducing a liquid polymer into the die cavity for forming the prosthetic lens and for flowing into at least a portion of the pores of the porous polymeric portion to form an integral solid portion and porous portion upon cooling of the liquid polymer.

2. A method as defined in claim 1, wherein the polymeric article is an ocular prosthesis and the porous portion forms a skirt (16) surrounding the lens (14) for permitting the ingrowth of living tissue into the pores of the skirt upon implantation in an eye.

3. A method as defined in claim 1, further comprising the step of compressing a portion of the porous portion upon introducing the liquid polymer into the die cavity (23) to permit the liquid polymer to flow into the pores of a limited area of the porous portion so that the remainder of the porous portion remains porous.

4. A method as defined in claim 2, wherein the liquid polymer is a polyether-based polyurethane.

5. A method as defined in claim 2, wherein the porous portion is made from a polymeric material selected from the group including a polyester and a polyether-based polyurethane.

6. A method as defined in claim 2, further comprising the step of cooling a surface defining the die cavity upon introducing the liquid polymer into the die cavity to facilitate obtaining high optical resolution with the lens.

7. A method as defined in claim 2, further comprising the step of heating the liquid polymer immediately prior to introduction into the die cavity to reduce the viscosity of the liquid polymer upon introduction into the die cavity.

8. A prosthesis for implantation within living tissue, comprising a solid polymeric portion forming a prosthetic lens (14) and an integral porous polymeric portion (16), formed by introducing the polymer of the solid portion as a heated liquid into the pores of at least a portion of the porous portion and permitting the liquid polymer to cool and simultaneously form the solid portion integral with the porous portion, the porous portion permitting the ingrowth of living tissue upon implantation.

9. A prosthesis as defined in claim 8, wherein the prosthesis is an ocular prosthesis and the porous portion forms a skirt (16) surrounding the lens (14) for permitting the ingrowth of living tissue into the pores of the skirt upon implantation in an eye.

10. A prosthesis as defined in claim 8, wherein the solid portion is made from a polyether-based polyurethane.

11. A prosthesis as defined in claim 8, wherein the porous portion is made from a material selected from the group including polyester and a polyether-based polyurethane.

12. An apparatus (10) for moulding a polymeric article (12) including a solid polymeric portion, said solid polymeric portion forming a prosthetic lens (14), and a porous polymeric portion (16), comprising:
a first die (20) for receiving said porous portion (16) of the article:
a second die (22) movable relative to the first die wherein the first and second dies define a die cavity (23) therebetween;
a means (38, 40) for conveying a liquid polymer to the cavity, the cavity forming said solid portion (14) of the article upon cooling of the liquid polymer within the die cavity and permitting a portion of the liquid polymer to flow into at least a portion (18) of the pores of the porous portion of the article to form an integral porous portion and solid portion upon cooling of the liquid polymer; and
a third die (24) movable relative to the first die and radially disposed about the second die for securing the porous portion of the article in position.

13. An apparatus as defined in claim 12, comprising a conduit (38) coupled in fluid communication with the die cavity for introducing the liquid polymer into the die cavity, and at least one heating element (42) located adjacent to a wall of the conduit for heating the liquid polymer immediately prior to introduction into the die cavity to reduce the viscosity of the liquid polymer upon introduction into the die cavity.

14. An apparatus as defined in claim 12, further comprising a cooling element (46) located adjacent to a surface defining the die cavity for cooling the surface to prevent the formation of flow lines and granularity on the surface of the solid portion of the article.

15. An apparatus as defined in claim 12, wherein the second die includes a surface (32) for compressing a portion of the porous portion to compress the pores and in turn substantially prevent the liquid polymer from flowing into that portion of the porous portion.

## Patentansprüche

1. Verfahren zum Formen eines Polymergegenstandes (12) mit einem kompakten Polymeranteil, wobei dieser feste Polymeranteil eine Protheselinse (14) bildet, und einem porösen Polymeranteil (16) mit den Stufen, in denen man den porösen Polymeranteil in einer Werkzeughöhlung (23) anordnet und ein flüssiges Polymer in die Höhlung zur Bildung der Protheselinse und zum Fließen in wenigstens einen Teil der Poren des porösen Polymeranteils unter Bildung eines einstückigen kompakten Anteils und porösen Anteils beim Kühlen des flüssigen Polymers einführt.

2. Verfahren nach Anspruch 1, bei dem der Polymergegenstand eine Augenprothese ist und der poröse Anteil eine Einfassung (16) bildet, die die Linse (14) umgibt, um das Einwachsen von lebendem Gewebe in die Poren der Einfassung nach Implantation in ein Auge zu erlauben.

3. Verfahren nach Anspruch 1 weiterhin mit der Stufe, in der man einen Teil des porösen Anteils bei Einführung des flüssigen Polymers in die Höhlung (23) komprimiert, um zu gestatten, daß flüssiges Polymer in die Poren eines begrenzten Bereiches des porösen Anteils fließt, so daß der Rest des porösen Anteils porös bleibt.

4. Verfahren nach Anspruch 2, bei dem das flüssige Polymer ein Polyurethan auf Polyetherbasis ist.

5. Verfahren nach Anspruch 2, bei dem der poröse Anteil aus einem Polymermaterial besteht, das aus der Gruppe ausgewählt ist, welche einen Polyester und ein Polyurethan auf Polyetherbasis einschließt.

6. Verfahren nach Anspruch 2, weiterhin mit der Stufe, in der man eine die Höhlung begrenzende Oberfläche bei Einführung des flüssigen Polymers in die Höhlung kühlt, um das Erhalten hoher optischer Auflösung mit der Linse zu erleichtern.

7. Verfahren nach Anspruch 2 weiterhin mit der Stufe, in der man das flüssige Polymer unmittelbar vor der Einführung in die Höhlung erhitzt, um die Viskosität des flüssigen Polymers bei Einführung in die Höhlung zu vermindern.

8. Prothese zur Implantation in lebendem Gewebe mit einem kompakten Polymeranteil, der eine Protheselinse (14) bildet, und einem mit ihm einstückigen porösen Polymeranteil (16), hergestellt durch Einführung des Polymers des kompakten Anteils als eine erhitzte Flüssigkeit in die Poren wenigstens eines Teils des porösen Anteils und Abkühlenlassen des flüssigen Polymers und gleichzeitige Bildung des kompakten Anteils einstückig mit dem porösen Anteil, wobei der poröse Anteil das Einwachsen von lebendem Gewebe nach der Implantation erlaubt.

9. Prothese nach Anspruch 8, wobei die Prothese eine Augenprothese ist und der poröse Anteil eine die Linse (14) umgebende Einfassung (16) bildet, um das Einwachsen von lebendem Gewebe in die Poren der Einfassung nach Implantation in einem Auge zu gestatten.

10. Prothese nach Anspruch 8, bei der der kompakte Anteil aus einem Polyurethan auf Polyetherbasis besteht.

11. Prothese nach Anspruch 8, bei der der poröse Anteil aus einem Material aus der Gruppe besteht, die Polyester und ein Polyurethan auf Polyetherbasis einschließt.

12. Vorrichtung (10) zum Formen eines Polymergegenstandes (12) mit einem kompakten Polymeranteil, wobei dieser kompakte Polymeranteil eine Protheselinse (14) bildet, und einem porösen Polymeranteil (16) mit
einem ersten Werkzeug (20) zur Aufnahme des porösen Anteils (16) des Gegenstandes,
einem zweiten Werkzeug (22), das in bezug auf das erste Werkzeug bewegbar ist, wobei das erste und das zweite Werkzeug eine Werkzeughöhlung (23) dazwischen begrenzen,
einer Einrichtung (38, 40) zur Beförderung eines flüssigen Polymers zu der Höhlung, wobei die Höhlung den kompakten Anteil (14) des Gegenstandes beim Kühlen des flüssigen Polymers in der Werkzeughöhlung bildet und erlaubt, daß ein Teil des flüssigen Polymers in wenigstens einen Teil (18) der Poren des porösen Anteils des Gegenstandes fließt, um einen einstückigen porösen Anteil und kompakten Anteil beim Kühlen des flüssigen Polymers zu bilden, und
einem dritten Werkzeug (24), das in bezug auf das erste Werkzeug bewegbar ist und radial um das zweite Werkzeug angeordnet ist, um den porösen Anteil des Gegenstandes in seiner Stellung festzulegen.

13. Vorrichtung nach Anspruch 12 mit einer Leitung (38) die in Fließverbindung mit der Höhlung gekoppelt ist, um das flüssige Polymer in die Höhlung einzuführen, und wenigstens einem Heizelement (42), das in Nachbarschaft zu einer Wand der Leitung angeordnet ist, um das flüssige Polymer unmittelbar vor der Einführung in die Werkzeughöhlung zu erhitzen und die Viskosität des flüssigen Polymers bei Einführung in die Werkzeughöhlung zu vermindern.

14. Vorrichtung nach Anspruch 12 weiterhin mit einem Kühlelement (46), das in Nachbarschaft zu einer die Werkzeughöhlung begrenzenden Oberfläche angeordnet ist, um die Oberfläche zu kühlen und die Bildung von Flußlinien und Körnigkeit auf der Oberfläche des kompakten Anteils des Gegenstandes zu verhindern.

15. Vorrichtung nach Anspruch 12, bei der das zweite Werkzeug eine Oberfläche (32) zum Komprimieren eines Teils des porösen Anteils einschließt, um die Poren zu komprimieren und seinerseits im wesentlichen zu verhindern, daß das flüssige Polymer in jenen Teil des porösen Anteils fließt.

## Revendications

1. Procédé de moulage d'un article polymère (12) incluant une partie polymère solide, ladite partie polymère solide formant une lentille prothétique (14), et une partie polymère poreuse (16), comprenant les étapes de placement de la partie polymère poreuse dans une cavité de matrice (23) et d'introduction d'un polymère liquide dans la cavité de matrice pour formage de la lentille prothétique et pour écoulement dans au moins une partie des pores de la partie polymère poreuse pour, après refroidissement du polymère liquide, former une partie solide et une partie poreuse d'un seul tenant.

2. Procédé selon la revendication 1, dans lequel l'article polymère est une prothèse oculaire et dans lequel la partie poreuse forme une jupe (16) entourant la lentille (14) pour, après implantation dans un oeil, permettre la croissance en profondeur de tissu vivant dans les pores de la jupe.

3. Procédé selon la revendication 1 comprenant, en outre, l'étape, après introduction du polymère liquide dans la cavité de matrice (23), de compression d'une partie de la partie poreuse pour permettre au polymère liquide de s'écouler dans les pores d'une zone limitée de la partie poreuse, de sorte que le restant de la partie poreuse reste poreux.

4. Procédé selon la revendication 2, dans lequel le polymère liquide est un polyuréthane à base de polyéther.

5. Procédé selon la revendication 2, dans lequel la partie poreuse est faite d'une matière polymère choisie à partir du groupe comprenant un polyester et un polyuréthane à base de polyéther.

6. Procédé selon la revendication 2 comprenant, en outre, l'étape, après introduction du polymère liquide dans la cavité de matrice, de refroidissement d'une surface définissant la cavité de matrice pour faciliter l'obtention d'une haute définition optique de la lentille.

7. Procédé selon la revendication 2 comprenant, en outre, l'étape, juste avant introduction dans la cavité de matrice, de chauffage du polymère liquide pour, après introduction dans la cavité de matrice, diminuer la viscosité du polymère liquide.

8. Prothèse pour implantation dans un tissu vivant, comprenant d'un seul tenant une partie polymère solide formant une lentille prothétique (14) et une partie polymère poreuse (16), formée en introduisant le polymère de la partie solide, sous forme de liquide chauffé, dans les pores d'au moins une partie de la partie poreuse et en permettant au polymère liquide de refroidir et de former simultanément la partie solide d'un seul tenant avec la partie poreuse, la partie poreuse permettant, après implantation, la croissance en profondeur de tissu vivant.

9. Prothèse selon la revendication 8, dans laquelle la prothèse est une prothèse oculaire et dans laquelle la partie poreuse forme une jupe (16) entourant la lentille (14) pour permettre, après implantation dans un oeil, la croissance en profondeur de tissu vivant dans les pores de la jupe.

10. Prothèse selon la revendication 8, dans laquelle la partie solide est faite d'un polyuréthane à base de polyéther.

11. Prothèse selon la revendication 8, dans laquelle la partie poreuse est faite d'une matière choisie à partir du groupe comprenant un polyester et un polyuréthane à base de polyéther.

12. Appareil (10) de moulage d'un article polymère (12) comprenant une partie polymère solide, ladite partie polymère solide formant une lentille prothétique (14) et une partie polymère poreuse (16), comprenant :
une première matrice (20) pour recevoir ladite partie poreuse (16) de l'article ;
une deuxième matrice (22) mobile par rapport à la première matrice, les première et deuxième matrices définissant, entre elles, une cavité de matrice (23) ;
un moyen (38, 40) pour amener un polymère liquide jusqu'à la cavité, la cavité formant, après refroidissement du polymère liquide dans la cavité de matrice, ladite partie solide (14) de l'article et pour permettre à une partie du polymère liquide de s'écouler dans au moins une partie (18) des pores de la partie poreuse de l'article pour, après refroidissement du polymère liquide, former une partie poreuse et une partie solide d'un seul tenant ; et
une troisième matrice (24) mobile par rapport à la première matrice et disposée de manière radiale autour de la deuxième matrice pour fixer, en position, la partie poreuse de l'article.

13. Appareil selon la revendication 12 comprenant un conduit (38) raccordé en communication fluidique à la cavité de matrice pour introduction du polymère liquide dans la cavité de matrice, et au moins un élément chauffant (42) situé au voisinage d'une paroi du conduit pour chauffage du polymère liquide juste avant introduction dans la cavité de matrice pour, après introduction dans la cavité de matrice, diminuer la viscosité du polymère liquide.

14. Appareil selon la revendication 12 comprenant, en outre, un élément refroidisseur (46) situé au voisinage d'une surface définissant la cavité de matrice pour refroidir la surface pour empêcher la formation de traces d'écoulement et de granularité sur la surface de la partie solide de l'article.

15. Appareil selon la revendication 12, dans lequel la deuxième matrice comprend une surface (32) de compression d'une partie de la partie poreuse pour comprimer les pores et, à son tour, empêcher sensiblement le polymère liquide de s'écouler dans cette partie de la partie poreuse.
